# EUROPEAN PATENT APPLICATION

(11) **EP 2 945 085 A1**
(43) Date of publication of application: **18.11.2015**
(21) Application number: 14382168.4
(22) Date of filing: 12.05.2014
(51) Int. Cl.: G06F 19/00

(54) **System and method for improving physical performance and control nutritional balance**

(71) Applicant: Rivero Jimenez, Angel, 28924 Madrid (ES); Munoz Holman, David, 28030 Madrid (ES); Martinez Serrano, Francisco Jesus, 28028 Madrid (ES)
(72) Inventor: Rivero Jimenez, Angel, 28924 Madrid (ES); Muñoz Holman, David, 28030 Madrid (ES)
(74) Representative: Lorca Melton, Miguel

(57) **Abstract**

The present invention is directed to a method for tracking the physical fitness of a mammal which comprises providing a body fluid sample to a biochip in order to obtain a first group of information, and processing said first group of information together with a second and third groups of information, said second group of information comprising biometric data of said mammal, and said third group of information comprising data of a specific physical activity performed by said mammal before providing the body fluid sample. The invention also provides a system to put into practice said method.

## Description

### FIELD OF THE INVENTION

The present invention belongs to the field of physical performance tracking by use of biochips for measuring analytes in body fluids samples, providing a fast and reliable reading thereof from the body fluid sample, and improve training and nutritional balance.

### PRIOR ART

Physical Fitness of an amateur or professional athlete is determined to a great extent by the quality an intensity of previous training. It is therefore critical to control the progression of the physical fitness, and to evaluate as soon as possible training results in order to be capable of reacting in case new strategies and/or diets are required. This is currently done by periodically performing physical tests under controlled conditions and also by tests such as body fluid analysis (e.g. blood, urine). Tracking the levels of certain analytes in body fluids provides an accurate and objective measurement of aspects of the athlete's physical fitness and the results of training.

Whether it is the case of a professional athlete seeking to improve the specific demands of his sport or an amateur wishing to improving health and fitness, it is always desirable to apply the correct and most efficient training method. Having periodic information on key analytes would dramatically improve self-knowledge and support decision making. In addition to intuitive knowledge, athletes would have objective data on how daily training is affecting their body, and could thus increase or decrease intensity, or even change completely training strategy, modulating the type, frequency or order of workouts. Such information would help in designing the correct periodization (i.e. order, intensity, nature and number of repetitions) of workouts and continuously obtaining biological data on the results, in order to further improve efficiency of future workouts in a virtuous cycle towards a more effective and personalised training.

It would also help maintaining accurate records and following the parameters of the prescribed workouts towards success of the training program. Therefore, a desire exists for an easy and efficient method of setting up, displaying, recording, and tracking workout information.

Many devices have been proposed or are being commercialised in order to track biological variables during sport training in order to monitor performance and provide suggestions for future workouts. For example, WO 2012/092221A1 to Basis Science Inc. describes a wrist watch capable of measuring different physiological parameters such as breathing, movement, heart rate or body temperature. This information is continuously monitored and stored in a computer readable expert system which continuously learns athletes routines and reactions, and is capable of suggesting training parameters based on an algorithm. Although it provides an improvement over previous sport tracking apparatus, it cannot provide data on body fluid biomarkers. Such information is of great value to evaluate progress and effectiveness of training. It has been demonstrated that exercise alters not only the levels of certain proteins but also changes some patterns of gene expression, or post translational modifications of muscle proteins. Therefore a frequent specific proteomic analysis might be a big change in the current training routines.

Current techniques to record analyte information in body fluids requires specialized laboratory work and is therefore expensive, slow and uncomfortable for the athlete, who has to provide a large amount of sample, send it to the laboratory and then wait for the results, days, or even weeks. The inconvenience of extracting body fluids, specially in the case of blood, only adds to the problem. These techniques are thus rarely used and are limited to elite athletes, and even in such cases only to a limited extent. Amateur athletes are thus prevented from using these techniques which would certainly help them improve results. The current situation is thus less than satisfactory, athletes having no means to easily track analytes on a periodic basis (e.g. daily or weekly) in an economic way. A further problem is the dependency on specialized personnel for taking samples and performing the analysis thereof. It will be desirable to provide apparatus and methods with simple and comfortable sample retrieval.

The field of microfluidics has been increasingly advancing in the last decades, and many apparatus exist with different configurations according to their use. For example, a number of devices have been proposed for analyzing blood samples. Depending on their morphology there are three types: (i) biosensors based on dry chemistry (e.g. based on glucose strips), such as those described in US 6,544,475; (ii) microarrays based on the diffusive static analysis on buffer liquids (e.g. microarrays for analysis of differential genetic expression - Richey, FPLC: A Comprehensive Separation Technique for Biopolymers, Am. Lab., 14:104 129 (1982)); and (iii) biochips based on the dynamic analysis in channels (e.g. Rong Fan et al, Nat Biotechnol. 2008 December; 26(12): 1373-1378).

The main advantage of biochips is their capacity to perform a direct analysis of body fluids without any prior treatment of the sample. Many biochips exist for analysing blood in the filed of medicine in the so called Points of Care (POC) or in doping detection. For example, WO 2013/043204 or US 2013/0078733 to Theranos Inc. disclose different aspects of POC systems and automatisation thereof.

Thus, there are currently in the market tracking systems for physical fitness, but they cannot provide real time information on key analytes present in the blood. Biochips exist which provide information of certain analytes but are not however suitable for tracking sport performance or physical fitness. They do not provide the information of a suitable set of analytes relating to sport performance or physical fitness and thus cannot support decision making in training strategies. Neither can it be found a system that processes information from key body fluid analytes in sport performance and physical fitness to track performance and support decisions on future training and diet habits. It would be desirable to provide a system to easily and reliably track analytes in body fluid samples, which would provide information on the physical fitness of the user and support the decision making process, in order to improve training strategies.

### BRIEF DESCRIPTION OF DRAWINGS

**Figure 1** provides an schematic view of the main units of an particular embodiment of a biochip according to the invention.
**Figure 2** provides an schematic flow chart of the expert system supporting the decision making features that can be applied to the data obtained by the tracking system of the invention.

### BRIEF DESCRIPTION OF THE INVENTION

It is the objective of the present invention to provide a system and methods that solve the above mentioned problems. Inventors have designed a system and method to track relevant analytes in body fluids samples (e.g. blood) through simple and economic means. The simple, economic and immediate information provided by the present invention provides perfect means for periodically tracking physical fitness through measuring key analytes, opening the door to daily evaluation of physical fitness through said analytes levels. Further, it is even economically and technically feasible to perform multiple analysis in the same day, giving the user, for example a professional or amateur athlete, the possibility of evaluating performance at different times and optimise his schedule. It is also possible with the means of the present invention to monitor the effect of diet on the user through information which is currently only available through costly and expensive laboratory techniques.

Thus a first aspect of the invention is the system defined in claim 1.

A further aspect of the invention is a method for tracking physical fitness as defined in claim 7.

A further aspect of the invention is the use of biochip for tracking the physical fitness and/or nutritional habits of a mammal.

A further aspect of the invention is the use of biochip for tracking physical fitness and/or nutritional habits and providing recommendations on further physical activity and/or further nutritional habits.

The present invention overcomes the problems of the prior art providing a fast, precise, portable, economic, non-invasive and flexible system and method to track physical fitness in order to support decision making towards improving training. It can be adapted for amateur and professional athletes, and it is specially indicated for gyms, personal training centres, high performance institutions, sports clubs (e.g. football, basketball), nutrition and cosmetic centres, and other training centres. It will help users improve their performance and increase motivation, compliance and will promote customer retention.

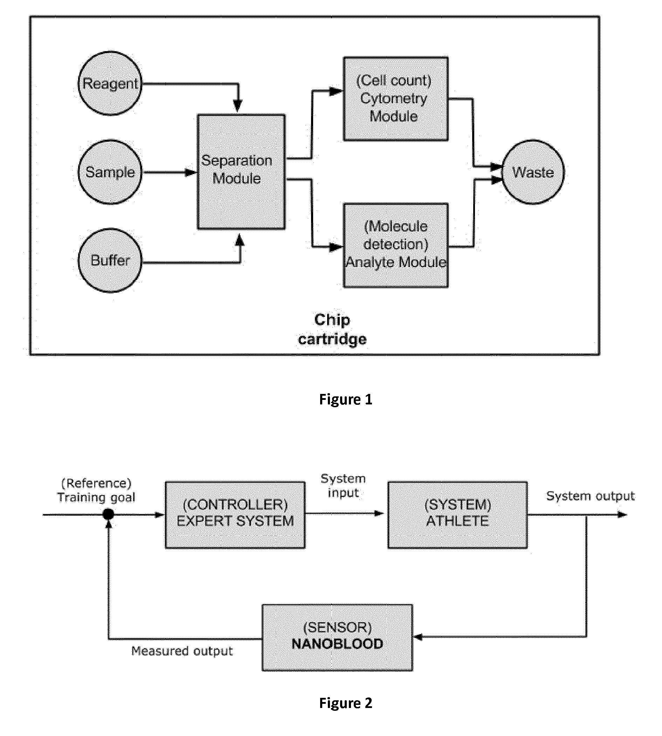

In an advantageous embodiment of the invention the processing unit comprises a computer readable expert system capable of processing the information from the biochip, the physical activity developed by the user, and his biometric data to provide one or more recommendations. Such computer readable expert systems have the additional advantage of being user friendly. The expert system's logic flow drives the user through different paths based on the knowledge base of the expert system and the answers provided by the user through the expert system's algorithms (e.g. see Figure 2).

Thus, there are other systems which may process biometric data, such as heart beat of a user, or even provide some recommendations based on the exercise performed by the user. However, the present invention provides a much more detailed analysis of the user's progress (e.g. objective biomolecular data on muscular progress) by being capable of processing, preferably through a computer readable expert system, biometric information of the user, information of each physical activity and, more importantly, objective information of key analytes in body fluid samples, which provide a picture of the user's development in unprecedented detail and simple fashion. This is provided by the invention without the need of an expert through a simple interface and display means of the information and recommendations.

### DETAILED DESCRIPTION OF THE INVENTION

Thus, the present invention makes use of biochips, devices having a small size capable of separating body fluids in different components and analyzing their content through different reactions. The biochips used in the present invention are preferably disposable, i.e. with limited number of uses, generally one. They are thus usually made of inexpensive materials to the extent possible, while being compatible with the reagents and the samples which are to be analyzed. In most instances, the biochips will be made of plastics such as polycarbonate, polystyrene, polyacrylates, or polyurethene, alternatively, they may be made from silicates, glass, wax or metal.

The biochips generally comprise an inlet port to receive the sample, in fluid connection with a plurality of channels and chambers which separate the components of the body fluid and contain the reagents and analyzers necessary to analyze the sample. The body fluid sample and its components are fluidly transported within the biochip by fluid means. The data resulting from the analysis is sent to an outlet port connected to a processing unit which processes and applies the algorithms to the data, and then display the resulting information in display means.

Biochips are generally small and flat, typically about 2 to 3 centimeters square or circular discs of similar size. Contrary to current analytic methods in the field of physical fitness, the volume of samples required is small e.g. 0.1 to 10 µL. It is thus specially indicated for continued use, for example, by athletes. There are several ways in which the capillaries and sample wells can be formed, such as injection molding, laser ablation, diamond milling or embossing. Generally, a base portion of the chip is cut to create the desired network of sample wells and capillaries and then a top portion will be attached over the base to complete the chip. They use small channels, chambers and fluid means to fluidly transport and separate different parts of body fluids to analyze them. For example, EP 1 579 191 A1 describes means for fluidly connecting body fluids to the different chambers of a biochip by means of capillarity.

The method and system of the invention may use any of the microfluidic devices that have been described in the art that are capable of separating body fluid components, preferably, blood components, provided with the suitable means for the analysis of analytes relevant for tracking physical fitness. For example, devices exist that are capable of carrying out separations based on size, for example, US 5,837,115; US 7,150,812; US 6,685,841 ; US 7,318,902; US 7,472,794; or US 7,735,652. Also, those described in US 8,053,201 are suitable.

Fluid means are capable of transporting the body fluid sample or some of its components through all the parts of the biochip, from the inlet port were the body fluid sample is deposited to the waste chamber. Exemplary non-limiting fluid means are capillarity, electrophoresis, electro-osmosis or pressure gradients using pumping, centrifugation or gravity. They must generate sufficient force to propel body fluid sample or its components through the channels. For example, the fluid means may be connected to a first chamber and create a force that is transmitted by fluid connection from said first chamber to a second chamber or other channels.

Biochips comprise at least one inlet port or sample port in fluid connection with separation means. Once the body fluid sample is separated, the components thereof are sent to one or more analyzers for optical or chemical analysis. Examples of types of analyzers that may be used, include flow cytometers, spectrophotometers, fluorescence detectors and radioactivity counters. The analyzer may be separate from the biochip but it is also possible for the analyzer to be integrated as part of the device itself. Preferably, the biochip has at least one port or opening. Finally, the biochip has a data output device for printing or displaying results from the analyzer. Usually this will be a computer or printer that displays the results of an optical or chemical analysis. The data output device may either be separate from, or part of, the analyzer.

The biochips of the present invention provide means for detecting different analytes. Analytes (also used here as synonym of biomarker) are understood as any biological molecule or structure (e.g. cells) that can be analyzed. According to a preferred embodiment, said analytes are one or more selected from the group consisting of glucose, albumin, creatinine, creatinine kinase, growth hormone (somatropin), lactate, urea, hemoglobin, ferritin, triglycerides, cholesterol, testosterone, cortisone, hematocrits, red cell count, white cell count and platelet count. The biochips of the invention are specially suitable to monitor sport performance. Due to the characteristics and functioning of biochips, all this can be done with only a drop of blood in the absence of specialized personnel.

The specific levels of these analytes in humans and other animals are the result of their training, genetic print, emotional situation, and environment, among others, and have a reflection in body response. By tracking such analytes we can track an individual's progress. The system and methods of the present invention allow monitoring effectively and periodically the concentration of such analytes. Other indicators such as maximum oxygen volume or body fat allow objective assessment of the physical fitness, but require special, costly and long tests in order to provide reliable results. The information provided by the method and apparatus of the present invention, including specific levels of chosen analytes, can allow the user or the trainer change periodicity and/or intensity of workouts. The present invention further provides an expert system capable of analyzing the data and suggesting workout routines according to the results obtained.

The system and method of the invention provides very specific information about the user, allowing for an extremely personalized monitoring and guidance for future workouts. It is possible to asses the impact in all or part of the analytes and changes in certain parameters, such as change in diet or intensity of workouts.

Overall, the continuous evolution of the analytes and thus of the physical fitness can be tracked over time. These continuous feedback at the same time allows the computer readable expert system to improve its decisions, improving the type of workouts, nutrition and other factors towards a specific objective, such as, for example, improving a specific physical fitness parameter (e.g. strength or resistance).

The method of the present invention can be carried out by means of a biochip capable of separating and analyzing different components of body fluids, preferably blood. The biochip may be housed in housing means to improve ergonomics and provide a more appealing appearance. It may comprise connection to the computer readable expert system capable of recording the data produced and compare it with standardized data. Further algorithms may even provide suggestions for further improvement. Data from the biochip can be loaded on a computer or other hand held devices such as smartphones, tablets and similar devices capable of running computer readable methods. All user information can be synced to a personal account over the web (cloud) to allow the user access his record from any device connected to the internet. The housing of the biochip can also comprise a control unit connected to display means in order to provide user with information about the analysis process.

The computer readable expert system may comprise a knowledge base and an inference machine configured to process said first, second and third groups of information in order to provide recommendations for future physical activity and/or nutrition habits. The knowledge base will comprise *inter alia* information regarding reference analyte levels for comparison, as well historical data from each user. The inference machine comprises the rules and algorithms which feed on the knowledge base and the data entered by the user, in order to provide recommendations for future physical activity. In an embodiment of the invention, the expert system may combine the information derived from the analysis of a given body fluid sample, preferably performed after a physical exercise, with historic data from the user, a proprietary database or a combination of both. In a further embodiment of the invention, the information processed to provide trends or patterns and provide advise on future physical exercise and/or diet habits.

### SPECIFIC EMBODIMENT

Figure 1 provides a schematic description of an exemplary biochip according to the present invention. In this example the athlete inserts a body part, e.g. a finger into a sample inlet, were a drop of blood is collected. The biochip comprises at least one separation module fluidly connected to said sample inlet, one or more reagent chambers and one or more buffer deposits. These elements are used to separate blood into predefined components. Two currents are formed, one rich in plasma and another rich in cells. These currents are allowed contact with the reagents in a synchronized way so as to provide the correct order and contact time for later detection. Some components are then fluidly transported to a cytometry module in order to provide a count of red and white cells, as well as platelets. Other components are transported to one or more analyte modules for detection of specific analyte levels.

The biochip comprises connection means configured to transfer the resulting data to a computer or other portable computing means, such as a smartphone or a tablet, say, for example, a smartphone. The smartphone includes a computer readable program for processing the input information provided by the user, and the data resulting from the biochip's analysis. Such computer readable program comprises an expert system which applies the predefined algorithms. The output of the algorithms include quantitative information, graphical display of different analyte levels and indicators, recommendations on diet and future workouts. Once the user has entered basic information regarding biometric parameters (e.g. age, hight, weight), data of each workout is entered, including, for example, intensity, periodization and duration. Once the analysis is complete, the results regarding the levels of the specific analytes are sent to the smartphone.

## Claims

1. Tracking system to monitor the physical fitness of a mammal comprising
a) one or more biochips capable of analysing from a body fluid sample provided thereto one or more analytes relevant to the physical fitness of said mammal and/or nutritional habits, so as to provide a first group of information;
b) means to provide a second and third groups of information, said second group of information comprising biometric data of said mammal, and said third group of information comprising data of a specific physical activity performed by said mammal before providing the body fluid sample;
c) means for transferring said first, second and third groups of information to a computer readable processing unit capable of processing said first, second and third groups of information; and
d) display means of at least one of said first, second and third groups of information.

2. Tracking system according to claims 1 comprising
a) one or more biochips capable of analysing from a body fluid sample one or more analytes relevant to the physical fitness of a mammal and/or nutritional habits, so as to provide a first group of information;
b) means to provide to the tracking system a second and third groups of information, said second group of information comprising biometric data of said mammal, and said third group of information comprising data of a specific physical activity performed by said mammal before providing the body fluid sample; and
c) means for transferring said first, second and third groups of information to a computer readable processing unit capable of processing and comparing said first, second and third groups of information in order to provide recommendations for future physical activity and/or nutrition habits
d) display means of at least one of said recommendations, said first group of information, said second group of information and said third group of information..

3. System according to any of claims 1 or 2, wherein said computer readable processing unit comprises a computer readable expert system, said computer readable expert system comprising a knowledge base and an inference machine configured to process said first, second and third groups of information in order to provide recommendations for future physical activity and/or nutrition habits.

4. System according to any of claims 1-3, wherein said one or more analytes are selected from the group consisting of glucose, albumin, creatinine, creatinine kinase, growth hormone, lactate, urea, hemoglobin, ferritin, triglycerides, cholesterol, testosterone, cortisone, hematocrits, red cell count, white cell count and platelet count.

5. System according to any of the previous claims, wherein said body fluid is blood.

6. System according to any of the previous claims wherein said third group of information comprises one or more selected from the group consisting of breathing information, heart rate information, body temperature, sweat, nutrition, number of exercises performed, type of exercises and effort perceived during and after the physical activity.

7. Method for tracking the physical fitness of a mammal which comprises providing a body fluid sample to a system as defined in any of the previous claims in order to obtain a first group of information, and processing said first group of information together with a second and third groups of information, said second group of information comprising biometric data of said mammal, and said third group of information comprising data of a specific physical activity performed by said mammal before providing the body fluid sample.

8. Method according to claim 7, which comprises providing a body fluid sample to a system as defined in any of the previous claims in order to obtain a first group of information, and comparing said first group of information with said second and third groups of information, by use of a computer readable expert system comprising (i) a knowledge base comprising information regarding reference analyte levels of body fluids and historical data from said mammal; and (ii) an inference machine comprising algorithms which feed on said knowledge base as well as on said first, second and third information groups, in order to provide recommendations for future physical activity and/or nutritional habits.

9. Method according to any of claims 7 or 8, wherein said first, second and third groups of information are processed and compared so as to provide recommendations on future physical activities and/or nutritional habits.

10. Method according to any of claims 7 to 9, wherein historical information on the physical fitness of the individual is also processed.

11. Method according to any of claims 7 to 10, for tracking sport performance in an athlete.

12. Use of a biochip for tracking the physical fitness and/or nutritional habits of a mammal.

13. Use according to claim 10 for tracking physical fitness after performing physical activity.

14. Use of a system as defined in any of claims 1 to 6 for tracking physical fitness and/or nutritional habits, and providing recommendations on further physical activity and/or further nutritional habits.
